# EUROPEAN PATENT APPLICATION

(11) **EP 3 772 326 A1**
(43) Date of publication of application: **10.02.2021**
(21) Application number: 19382687.2
(22) Date of filing: 06.08.2019
(51) Int. Cl.: A61B 5/00

(54) **A DERMATOSCOPY DEVICE AND A METHOD FOR CHECKING SKIN LESIONS**

(71) Applicant: DERMAVISION SOLUTIONS, SL, 17480 Roses (Girona) (ES)
(72) Inventor: CAMPMOL AMETLLER, Enric, 17480 Roses (Girona) (ES); RICART GELI, Narcís, 17480 Roses (Girona) (ES)
(74) Representative: Juncosa Miró, Jaime

(57) **Abstract**

A dermatoscopy device and a method for checking skin lesions are disclosed. The device comprises an enclosed chamber (10); a displacement element (12) to be moved inside the chamber (10); an image acquisition system (20) attached to the displacement element (12); lighting elements (13, 14) and a computing unit (30). The image acquisition system (20) has a stereoscopic camera (21, 22) and a dermatoscopic camera (23). The computing unit (30) analyzes, for each body region of the user, an image acquired by the stereoscopic camera (21, 22); executes a scanning algorithm that detects skin structures in said image and that checks whether said structures fulfill a criteria; upon a given skin structure is determined as fulfilling the criteria, executes a motion algorithm that moves the displacement element (12) to said given body region; and triggers an order, in order the dermatoscopic camera (23) acquiring, at a determined distance, an image of said skin structure.

## Description

### Technical Field

The present invention is directed, in general, to the dermatology field. In particular, the invention relates to a dermatoscopy device, and to a method, for checking skin lesions.

### Background of the Invention

International patent application WO-A1-2013169326 discloses an imaging station/booth for automated total body imaging having a small footprint and capable of quickly, efficiently, effectively, and consistently capturing multiple body images of a user or patient over time with minimal assistance from medical staff.

International patent application WO-A1-2018160720 relates to three-dimensional image capture, particularly with the use of polarized light. The capture of three-dimensional (3D) images of a subject, such as a human, typically entails the use of multiple cameras and light sources, arranged around the subject, that are activated simultaneously while the subject remains still. The number of cameras and light sources required depends in direct relation on the size of the area to be imaged.

International patent application WO-A1-2010107467 discloses an imaging station and method for repeatable alignment of images. In some embodiments, the imaging station includes a rotatable stage and a monostand. The rotatable stage may include a structure to support sets of handles for positioning of a subject's hands for at least some of the body poses. A camera is positioned on the monostand for capturing images of the subject and is configured to allow the camera to be repeatably positioned with respect to the rotatable stage for each body pose.

US patent US-B1-9980649 discloses a scan head for scanning skin. The scan head includes a frame and a camera coupled to the frame. A controllable probe is coupled to the frame and is configured to change an orientation of hair on the skin to be examined and imaged with the camera.

International patent application WO-A1-2016188659 discloses a method and a device for producing a series of images of an object with respective predetermined imaging parameters by means of a partially automated imaging system, comprising a database, an imaging unit, a computing unit, a storage unit and an output unit, wherein it is ensured that a manually to be adjusted imaging parameter corresponds to an actual imaging parameter.

In the known dermatoscopy imaging devices/stations, a single dermatoscopic image is acquired invasively, i.e. not acquired from a distance at least greater than 1-3 mm. Moreover, the known imaging devices/stations do not allow operating in fully controlled lighting conditions using a single image acquisition system comprising different types of cameras.

New imaging devices and method for checking skin lesions in a location with controlled light and image acquisition are therefore needed.

### Description of the Invention

To that end the present invention proposes, according to one aspect, a dermatoscopy device for checking skin lesions.

Similar to the devices known in the field, the proposed dermatoscopy device comprises:
- an enclosed chamber to accommodate a user/patient to be examined;
- a displacement element to be moved on axes x, y, z inside the enclosed chamber;
- an image acquisition system attached on a support of the displacement element in order images of the user being acquired from different body regions of said user;
- lighting elements;
- a computing unit, operatively connected to said displacement element and to said image acquisition system, to process said acquired images.

Unlike the known devices in the field, the image acquisition system of the proposed device comprises at least one stereoscopic camera and a dermatoscopic camera (e.g. a long-range dermatoscopic camera). The at least one stereoscopic camera is configured to operate independently of the dermatoscopic camera. Besides, the lighting elements comprise at least two lighting elements each one attached on an extreme of the support of the displacement element. Thus, in the proposed device, the images are always acquired under controlled lighting conditions.

Moreover, in the proposed device the cited processing comprises analyzing, for each body region, at least one image acquired by the at least one stereoscopic camera; executing a scanning algorithm that detects skin structures (e.g. freckles, skin marks, among others) in said image and that checks whether said skin structures fulfill a given criteria; upon a given skin structure of a given body region being determined as fulfilling the given criteria, executing a motion algorithm that automatically moves the displacement element to said given body region; and providing a triggering order to the image acquisition system in order the dermatoscopic camera acquiring, at a determined distance, at least one image of said given skin structure.

Therefore, present invention provides a medical device that provides automated and accurate clinical and dermatoscopic images of a patient's skin. Moreover, the dermatoscopic images of individual skin lesions are acquired in a standardized way (controlled light and acquisition distance). In addition, the present invention provides the physician/doctor with the necessary information such as the color, dimension, shape, asymmetry and evolution of each of the skin structures in order to make an accurate diagnosis.

In an embodiment, the image acquisition system comprises two stereoscopic cameras (or two cameras configured to work as a stereoscopic camera). The two stereoscopic cameras are configured to operate simultaneously and independently of the dermatoscopic camera. Alternatively, the stereoscopic camera is a structured-light 3D sensor, a time-of-flight (TOF) camera or the combination of a LIDAR and a single camera.

In an embodiment, said determined distance is comprised in a range between 100-1000 mm from the user, preferably between 300-600 mm. Therefore, the acquisition is performed non-invasively.

In an embodiment, the displacement element is arranged on a guiding railway of a guiding system. The guiding system is arranged on an interior wall of the enclosed chamber. Particularly, the guiding system comprises two vertical guide members.

In another embodiment, each of the lighting elements comprises a light emitting diode (LED). The lighting elements also include a light confinement chamber in order to only illuminate a given zone when the image/s is/are acquired. Furthermore, the lighting elements also include a polarization filter, which is 90° out of phase with respect to a polarization filter of the dermatoscopic camera in order to generate a cross polarization.

The lighting elements are separated a certain distance on said support in order to operate at a given angle (between 30 and 45º).

In yet another embodiment, the proposed device also has a positioning unit to indicate a position in which the user has to be placed inside the enclosed chamber. For example, the position unit can be a floor display or even a loudspeaker which guides the user to the different positions. Alternatively, the positioning unit can be configured to automatically move the user towards said interior wall of the enclosed chamber.

Embodiments of the present invention also propose, according to another aspect, a method for checking skin lesions. The method comprises a) accommodating a user to be examined within an enclosed chamber of a dermatoscopy device; b) moving, via a displacement element of the dermatoscopy device, an image acquisition system of said dermatoscopy device towards different body parts of a given body side of the user; c) acquiring, by at least one stereoscopic camera of said image acquisition system, one or more stereoscopic images of said different body parts of the user, wherein the one or more stereoscopic images are acquired under controlled lighting conditions; d) analyzing, by a computing unit, the acquired stereoscopic images by executing a scanning algorithm that checks whether skin structures included in said stereoscopic images fulfill a given criteria; e) upon a given skin structure of a given body region is determined as fulfilling the given criteria, executing, by the computing unit, a motion algorithm that automatically moves the displacement element towards said given body region; f) in response to a triggering order from the computing unit, acquiring, by a dermatoscopic camera of the image acquisition system, at a determined distance, at least one dermatoscopic image of said given skin structure; and g) providing the acquired at least one dermatoscopic image using the computing unit.

According to the proposed method, said step b) is performed for all the body sides of the user.

In an embodiment, the cited criteria is based on an ABCD rule of dermatoscopy based on the criteria asymmetry, A, border, B, color, C, and differential structure, D, of the skin structures.

In another embodiment, the scanning algorithm comprises a neural network. The neural network is trained to automatically recognize if the skin structure looks like a benign or malign structure.

### Brief Description of the Drawings

The previous and other advantages and features will be more fully understood from the following detailed description of embodiments, with reference to the attached figures, which must be considered in an illustrative and non-limiting manner, in which:
Fig. 1 illustrates the proposed dermatoscopy device for checking skin lesions, according to an embodiment of the present invention.
Fig. 2 illustrates in more detail the image acquisition system, lighting elements and displacement element of the embodiment of Fig. 1.
Fig. 3 is a flow chart showing the steps executed by the computing unit to check skin lesions, according to an embodiment of the present invention.

### Detailed Description of Preferred Embodiments

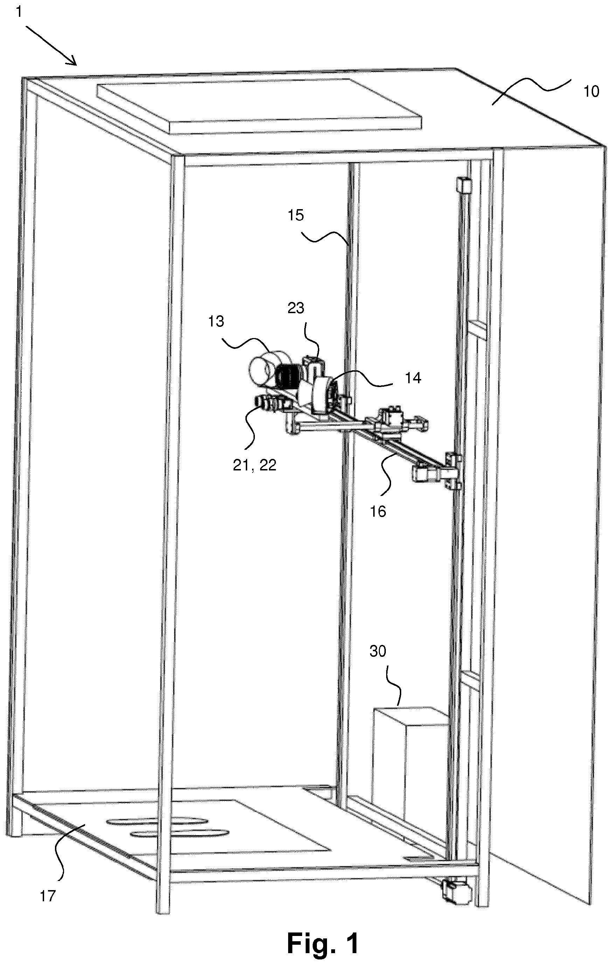

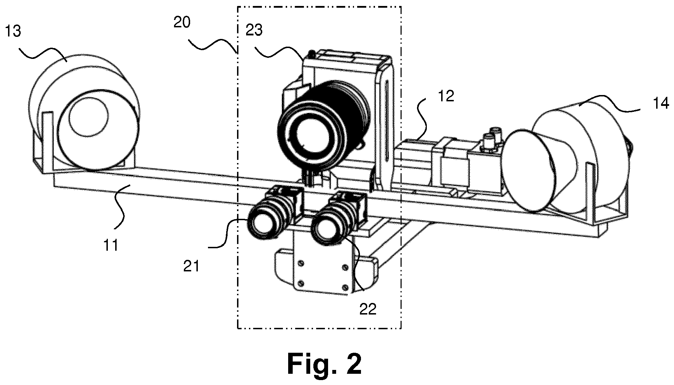

Figs. 1 and 2 show the proposed architecture of the invention for checking skin lesions, including primary and secondary skin lesions, according to an embodiment. In this particular embodiment, the dermatoscopy device 1 includes an enclosed chamber 10 designed to accommodate a user/patient to be examined; a guiding system 15 formed by two vertical guide members attached on an interior wall of the enclosed chamber 10 (not limitative as in other embodiments the guiding system can comprise a robotic arm or can be formed by guide members attached on different interior walls of the enclosed chamber 10); a displacement element 12 arranged on a guiding railway 16 of the cited guiding system 15 and suitable to be moved on the three axes (x, y, z) inside the enclosed chamber 10; an image acquisition system 20 attached on a support 11 of the displacement element 12; two lighting elements 13, 14; a positioning unit 17 and a computing unit 30 with one or more processors and at least one memory.

The computing unit is operatively connected to the displacement element 12 and to the image acquisition system 20 and is adapted and configured to implement/execute different algorithms in order to control operation thereof. It should be noted that in other embodiments, the computing unit 30 instead of being included in the dermatoscopy device 1 can be located remote from it. For example, in this latter case, the computing unit can be a PC, a cloud server, a Tablet, etc. placed distant from the dermatoscopy device 1.

In the particular embodiment of Figs. 1 and 2, the image acquisition system 20 includes two cameras 21, 22 that are configured to operate as a stereoscopic camera (hence from now on being termed stereoscopic cameras) and a dermatoscopic camera 23. The two stereoscopic cameras 21, 22 operate simultaneously and regardless of the dermatoscopic camera 23. The dermatoscopic camera 23 is configured to acquire dermatoscopic images at a certain distance from the user, for example, at 100-800 mm, preferably at 300-500 mm from the user.

In other embodiments (not showed), the image acquisition system 20 includes a single stereoscopic camera consisting of a structured-light 3D sensor.

With regard to the two lighting elements 13, 14, each one comprises a high-power LED, with a light confinement chamber and a refrigerating system (i.e. each LED element has a radiator and a fan attached). Moreover, each lighting element 13, 14 has a polarization filter having a 90°offset with respect to a polarization filter of the dermatoscopic camera 23. Thus, a cross polarization is generated.

The two lighting elements 13, 14 are arranged on the cited support 11 separated from the image acquisition system 20 in order to operate at a given angle (between 30 and 45º) considering the operating distance of the dermatoscopic camera 23.

In the embodiment of Figs. 1 and 2, the positioning unit 17 comprises a floor display that is configured to indicate to the user a position in which (s)he has to be placed/located inside the enclosed chamber in order the images from different body sides being acquired. In this case, the user has to move herself/himself to the indicated position. In other embodiments, the positioning unit 17 can automatically move the user towards the displacement element and image acquisition system 20.

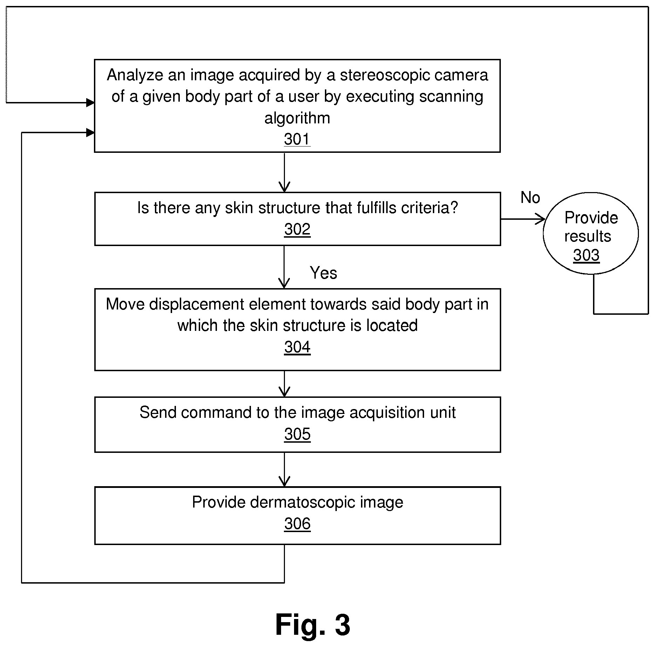

With reference now to Fig. 3, therein it is illustrated an embodiment of the processing steps executed by the computing unit. This method is initiated once a user to be examined is accommodated within the enclosed chamber 10 of the proposed dermatoscopy device 1 and once the image acquisition system 20 is moved towards a first body part (e.g. the torso) of a given body side (e.g. the ventral side of the body) of the user. In this case it has been supposed that the image acquisition system 20 includes a structured-light 3D sensor and a dermatoscopic camera.

At step 301, the computing unit analyzes the acquired stereoscopic image(s) by executing a scanning algorithm that checks whether skin structures (e.g. actinic keratosis, moles, skin marks, etc.) included in said stereoscopic image(s) fulfill a given criteria (step 302). If no skin structures fulfilling the criteria are found, the computing unit simply provides (step 303) the results of the previous analysis. Otherwise, i.e. if a given skin structure has been determined as fulfilling the given criteria, the computing unit, at step 304, executes a motion algorithm that automatically moves the displacement element 12, and so the image acquisition system 20 and the lighting elements 13, 14, towards the body part where the skin structure is located. Then, at step 305, the computing unit triggers a command/order to the image acquisition unit 20 in order the dermatoscopic camera 23 acquiring, at a determined distance from the user, particularly at approx. 300-600 mm from the user, at least one dermatoscopic image of said skin structure. Once the dermatoscopic image is acquired, the computing unit, at step 306, provides the dermatoscopic image, for example via a display thereof, via a specific user interface, by printing it, etc. In this sense, a physician/doctor can latter use the dermatoscopic image to make an accurate diagnosis of the skin lesion.

It should be noted that, previous steps are performed for all the body sides of the user, if necessary. For example, for the lateral and dorsal sides of the user, and for different body parts, such as the legs, arms, head, back, etc., of each body side. If the user has to be moved to a given position in order the image/images being acquired from a specific body part or body side, a further step of the method could be an indication of such a position.

In certain embodiments, said criteria is based on an ABCD rule of dermatoscopy based on the criteria asymmetry, A, border, B, color, C, and differential structure, D, of the skin structures. Complementarily or alternatively, in certain embodiments, the scanning algorithm may comprise a neural network.

In other embodiments, in the particular case that two stereoscopic cameras 21, 22 configured to operate as a stereoscopic camera are used, the image(s) acquired by each camera are analyzed in parallel. Once the scanning algorithm has detected a skin structure in one of the stereoscopic images, it matches the position thereof in the other stereoscopic image in order to identify the exact position of the skin structure.

Those skilled in the art will recognize that the present teachings are amenable to a variety of modifications and/or enhancements. All applications, modifications and alterations required to be protected in the claims may be within the protection scope of the present disclosure.

The scope of the present invention is defined in the following set of claims.

## Claims

1. A dermatoscopy device for checking skin lesions, comprising:
an enclosed chamber (10) to accommodate a user to be examined;
a displacement element (12) configured to be moved on axes x, y, z inside the enclosed chamber (10);
an image acquisition system (20) attached on a support (11) of the displacement element (12) in order images of the user being acquired from different body regions of said user;
lighting elements (13, 14); and
a computing unit (30), operatively connected to said displacement element (12) and to said image acquisition system (20), and configured to process said acquired images;
**characterized in that:**
said image acquisition system (20) comprises at least one stereoscopic camera (21, 22) and a dermatoscopic camera (23), wherein said at least one stereoscopic camera (21, 22) is configured to operate independently of the dermatoscopic camera (23);
said lighting elements (13, 14) comprises at least two lighting elements each one attached on an extreme of the support (11) of the displacement element (12); and
said processing comprises:
• analyzing, for each body region, at least one image acquired by the at least one stereoscopic camera (21, 22);
• executing a scanning algorithm that detects skin structures in said image and that checks whether said skin structures fulfill a given criteria;
• upon a given skin structure of a given body region being determined as fulfilling the given criteria, executing a motion algorithm that automatically moves the displacement element (12) to said given body region; and
• providing a triggering order to the image acquisition system (20) in order the dermatoscopic camera (23) acquiring, at a determined distance, at least one image of said given skin structure.

2. The device of claim 1, wherein the image acquisition system (20) comprises two stereoscopic cameras (21, 22) configured to operate simultaneously and independently of the dermatoscopic camera (23).

3. The device of claim 1, wherein the stereoscopic camera is a structured-light 3D sensor, a time-of-flight, TOF, camera or the combination of a LIDAR and a single camera.

4. The device of any of previous claims, wherein the displacement element (12) is arranged on a guiding railway (16) of a guiding system (15), the guiding system (15) being arranged on an interior wall of the enclosed chamber (10).

5. The device of any of previous claims, wherein each of the lighting elements (13, 14) comprises a light emitting diode, LED, with a light confinement chamber and a polarization filter, wherein said polarization filter has a 90°offset with respect to a polarization filter of the dermatoscopic camera (23).

6. The device of any of previous claims, further comprising a positioning unit (17) configured to indicate a position in which the user has to be placed inside the enclosed chamber (10).

7. The device of claim 4, further comprising a positioning unit (17) configured to automatically move the user towards said interior wall of the enclosed chamber (10).

8. The device of any of previous claims, wherein said criteria is based on an ABCD rule of dermatoscopy based on the criteria asymmetry, A, border, B, color, C, and differential structure, D, of the skin structures.

9. The device of claim 1, wherein said distance is comprised in a range between 100-1000 mm from the user, preferably between 300-600 mm.

10. A method for checking skin lesions, comprising:
a) accommodating a user to be examined within an enclosed chamber (10) of a dermatoscopy device (1);
b) moving, via a displacement element (12) of the dermatoscopy device (1), an image acquisition system (20) of said dermatoscopy device (1) towards different body parts of a given body side of the user;
c) acquiring, by each at least one stereoscopic camera (21, 22) of said image acquisition system (20), one or more stereoscopic images of said different body parts of the user, wherein the one or more stereoscopic images being acquired under controlled lighting conditions;
d) analyzing, by a computing unit (30), the acquired stereoscopic images by executing a scanning algorithm that checks whether skin structures included in said stereoscopic images fulfill a given criteria;
e) upon a given skin structure of a given body region being determined as fulfilling the given criteria, executing, by the computing unit (30), a motion algorithm that automatically moves the displacement element (12) towards said given body region;
f) in response to a triggering order from the computing unit (30), acquiring, by a dermatoscopic camera (23) of the image acquisition system (20), at a determined distance, at least one dermatoscopic image of said given skin structure; and
g) providing the acquired at least one dermatoscopic image using the computing unit (30),
wherein said step b) being performed for all the body sides of the user.

11. The method of claim 10, wherein said criteria is based on an ABCD rule of dermatoscopy based on the criteria asymmetry, A, border, B, color, C, and differential structure, D, of the skin structures.

12. The method of claim 10, wherein the scanning algorithm comprises a neural network.

13. The method of claim 10, wherein previously to said step b), a positioning unit (17) of the dermatoscopy device (1) indicates a position in which the user has to be placed inside the enclosed chamber (10).

14. The method of claim 10, wherein previously to said step b), a positioning unit (17) automatically moves the user towards an interior wall of the enclosed chamber (10), the latter having arranged therein a guiding system (15), the guiding system (15) comprising a guiding railway (16) through which the displacement element (12) moves.

15. The method of claim 10, wherein said distance is comprised in a range between 1000-800 mm from the user, preferably between 300-600 mm.
